# EUROPÄISCHE PATENTSCHRIFT

(11) **EP 0 909 753 B1**
(45) Veröffentlichungstag und Bekanntmachung des Hinweises auf die Patenterteilung: **14.01.2004**
(21) Anmeldenummer: 98119367.5
(22) Anmeldetag: 14.10.1998
(51) Int. Cl.: C07C 213/10, C07C 215/44, C07B 63/00

(54) **Verfahren zur Herstellung von trans-4-Aminocyclohexanol**
Process for the preparation of trans-4-aminocyclohexanol
Procédé pour la préparation du trans-amino-4-cyclohexanol

(30) Priorität: 15.10.1997 DE 19745529
(43) Veröffentlichungstag der Anmeldung: 21.04.1999
(73) Patentinhaber: Lehmann, Bernd, Dr., 78465 Konstanz (DE)
(72) Erfinder: Lehmann, Bernd, Dr., 78465 Konstanz (DE)

(56) Entgegenhaltungen:
- GB-A- 454 042
- GB-A- 2 239 242
- US-A- 2 152 960
- DATABASE WPI Section Ch, Week 197748 Derwent Publications Ltd., London, GB; Class A60, AN 1977-86135Y XP002145265 & SU 550 379 A (MONOMERS RES INST ET AL.), 27. April 1977 (1977-04-27)
- ERWIN FERBER ET AL.: "Die Synthese der 4-Aminocyclohexyl-(1)-methylketone." BERICHTE DER DEUTSCHEN CHEMISCHEN GESELLSCHAFT., Bd. 72, Nr. 5, 1939, Seiten 995-1002, XP002145262 WEINHEIM DE
- JOHN H. BILLMAN ET AL.: "Amino acids." JOURNAL OF THE AMERICAN CHEMICAL SOCIETY., Bd. 75, Nr. 5, 10. März 1953 (1953-03-10), Seiten 1345-1346, XP002145263 DC US
- CHEMICAL ABSTRACTS, vol. 73, no. 9, 31. August 1970 (1970-08-31) Columbus, Ohio, US; abstract no. 45244f, FRASER ROBERT R. ET AL.: "Synthesis of 7-azabicyclo(2.2.1) heptane,..." Seite 298; Spalte 2; XP002145264 & CANA. J. CHEM., Bd. 48, Nr. 13, 1970, Seiten 2065-2074,

## Beschreibung

Die Erfindung betrifft ein Verfahren zur Herstellung von trans-4-Aminocyclohexanol. Diese Verbindung ist eine wichtige Zwischenverbindung bei der Herstellung von Arzneimitteln (z. B. Ambroxol, trans-4-(2-Amino-3,5-dibrombenzylamino)cyclohexanol).

Im Stand der Technik gibt es eine Reihe von Verfahren, die zum trans-Aminocyclohexanol führen. Häufig geht man von Paracetamol (p-Acetamido-phenol) aus, das in wäßriger oder alkoholischer Lösung katalytisch hydriert wird. Eine Isomerentrennung findet dann über eine fraktionierte Kristallisation des Acetamidocyclohexanols aus Aceton statt. Die freien Aminoalkohole erhält man dann durch Hydrolyse des isolierten, getrockneten trans-Acetamidocyclohexanols, beispielsweise durch Umsetzung im sauren und/oder alkalischen Milieu unter Freisetzung der Aminofunktion (d. h. Abspaltung von Essigsäure). Das Endprodukt wird durch Extraktion mit Chloroform aus der alkalischen bzw. alkalisierten Reaktionslösung erhalten. Diese Verfahrensweise ist mit einer niedrigen Ausbeute verbunden und zudem aufwendig (vgl. Ferber und Brückner in Chem.Ber., 72, 1939, Seiten 995-1002).

Eine Isomerentrennung aus einem Gemisch von trans- und cis-4-Aminocyclohexanol wird im Zusammenhang mit diesen Verfahren im Stand der Technik nicht beschrieben. Auch andere Umsetzungsstrategien, bei denen man von trans-Cyclohexanolvorläufern der Zielverbindung ausgeht (vgl. beispielsweise R.R. Fraser, R.B. Swingle in Can.J.Chem. 48, 1970, Seiten 2065-2074) erwägen an keiner Stelle die Isomerentrennung auf der Stufe der 4-Aminocyclohexanole, d. h. z. B. deren selektive Abtrennung/Kristallisation aus wäßrigen Lösungen.

Möglicherweise hat der Fachmann von dieser zumindest theoretisch bestehenden Möglichkeit Abstand genommen, da in jedem Fall bei der notwendigen Hydrolyse der Vorläuferstufen ein wäßriges Lösemittel zum Einsatz kommt und zum anderen die Aminocyclohexanole eine sehr gute Löslichkeit in einem solchen Lösemittel haben.

Aus dem Derwent Abstract Nr. 77-86135 Y/48 ist die Trennung der Stereoisomeren von Aminocyclohexanol durch wiederholte Umkristallisation aus Ethylenglycoldimethylether unter Abkühlen auf 0-15°C bekannt. Diese Vorgehensweise wird erfindungsgemäß nicht beansprucht.

Denkbar wäre theoretisch auch die unmittelbare Hydrierung von Aminophenolen und anschließende Aufarbeitung des Isomerengemisches durch fraktionierte Kristallisation oder andere Trennverfahren. Allerdings wird in dem britischen Patent 454 042 berichtet, daß ausgehend von para-Aminophenol keinerlei para-Aminocyclohexanol erhalten bzw. isoliert werden konnte. Ferber und Brückner (siehe oben) berichten, daß die Hydroxylgruppe des p-Aminophenols labil ist und deshalb während der Hydrierung leicht abgespalten wird. Lediglich bei L.Kh. Freidlin, et al. in Bull.Acad.Sci. USSR Div.Chem.Sci., 25, 1976, Seiten 952-958) wird über die erfolgreiche Hydrierung von p-Aminophenol in einem wäßrigen System mit einem Ruthenium/Al₂O₃-Katalysator berichtet. Bei dieser Umsetzung werden aber 86 % des cis- und nur 14 % des trans-Produktes erhalten. Der Isomerengehalt wurde in dieser Arbeit säulenchromatographisch bestimmt. Über eine tatsächliche Isomerentrennung wird nicht berichtet. Das Verfahren dieser Druckschrift wäre zudem für eine vernünftige und wirtschaftliche Darstellung von trans-Aminocyclohexanol nicht geeignet, da überwiegend das nicht erwünschte cis-Produkt erhalten wird (86 %) .

Es besteht somit ein erhebliches Bedürfnis nach einem einfachen Verfahren zur Herstellung von trans-Aminocyclohexanol mit dem sich eine Isomerentrennung leicht und insbesondere mit hoher Ausbeute durchführen läßt. Weiterhin besteht ein Bedürfnis nach einem einfachen Gesamtverfahren unter Einbeziehung einer Hydrierung von Paracetamol (p-Acetaminophenol).

Um dieses Ziel zu erreichen, reicht es aus, die folgenden Bedingungen zu erfüllen:
1. Der Gefrierpunkt einer die Isomeren trans-4-Aminocyclohexanol und cis-4-Aminocyclohexanol enthaltenden wäßrigen Lösung wird auf mindestens -5°C (unter Normalbedingungen) eingestellt;
2. das Verhältnis trans-4-Aminocyclohexanol zu cis-4-Aminocyclohexanol beträgt mindestens 1 : 1, vorzugsweise 4 : 1 und darüber;
3. Abkühlung der Lösung bis auf eine Temperatur, bei der das trans-4-Aminocyclohexanol ausfällt/auskristallisiert.

Der Erfinder hat überraschenderweise gefunden, daß bei Einhaltung dieser Bedingungen selektiv trans-Aminocyclohexanol in hoher Reinheit ausfällt. Eine Gefrierpunktserniedrigung auf einen Wert von mindestens -5°C kann man auf verschiedene Weise erreichen. Beispielsweise hat bereits das Aminocyclohexanol selber eine gefrierpunktserniedrigende Wirkung. Folglich bestimmt auch die Konzentration des Aminocyclohexanols in der wässrigen Lösung den Gefrierpunkt. Weiterhin ist es möglich, durch Zusatz anderer salzartiger Verbindungen den Gefrierpunkt herabzusetzen. Es hat sich als praktikabel erwiesen, die Gefrierpunktserniedrigung im wesentlichen durch Zugabe von KOH und/oder NaOH, beispielsweise Kali- und/oder Natronlauge, einzustellen. Vorzugsweise wird der Gefrierpunkt der Lösung auf einen Wert von -10°C eingestellt. Jedenfalls sollte die Gefrierpunktserniedrigung soweit getrieben werden, daß sie unterhalb der Temperatur liegt, bei der das trans-Aminocyclohexanol ausfällt. Diese Temperatur liegt regelmäßig bei etwa -8°C, sie kann aber auch deutlich nach oben oder unten von diesem Wert abweichen. Die jeweiligen optimalen Verfahrensbedingungen kann ein Fachmann im Rahmen einfacher Versuche leicht feststellen. Bei ungünstigeren Isomerenverhältnissen, beispielsweise 1 : 1 kann es erforderlich sein, weitere Umkristallisationsstufen anzuschließen. Es hat sich aber gezeigt, daß bei einem Isomerenverhältnis (trans/cis) von mindestens 3 : 1 die trans-Verbindung nahezu vollständig

Der Ausdruck "wäßrige Lösung" bedeutet im Rahmen der vorliegenden Erfindung, daß das Lösemittel im wesentlichen aus Wasser besteht. Es können aber auch andere Co-Lösemittel vorhanden sein, wie beispielsweise Alkohole. Es können bis zu 10 % Methanol oder Ethanol vorhanden sein.

Gegenstand der Erfindung ist folglich ein Verfahren zur Isomerentrennung einer Mischung aus trans-4-Aminocyclohexanol und cis-Aminohexanol, dadurch gekennzeichnet, daß
a) eine Isomerenmischung aus trans-4-Aminocyclohexanol und cis-4-Aminocyclohexanol mit einem trans/cis-Verhältnis von mindestens 1 : 1 eingesetzt wird,
b) in einer wäßrigen Lösung dieser Mischung so viel KOH oder NaOH (Kalilauge/Natronlauge) ein- bzw. zugesetzt wird, daß der Gefrierpunkt auf mindestens -5°C absinkt, und
c) unter Abkühlung der wässrigen Lösung trans-4-Aminocyclohexanol auskristallisiert wird.

Eine wäßrige Lösung, die den oben angegebenen Bedingungen genügt, hat einen pH-Wert von mindestens 13,5, regelmäßig deutlich darüber.

Ganz besonders glatt gelingt das erfindungsgemäße Verfahren, wenn die Ausgangslösung mindestens 0,5 - 3 Mol/l p-Aminocyclohexanol (aber auch darüber) und mindestens 2 - 4 Mol/l OH⁻ Ionen in der wäßrigen Lösung vorhanden sind. Vorzugsweise fällt das trans-Aminocyclohexanol bei etwa -8°C aus.

Zur Einstellung der geeigneten OH⁻-Ionenkonzentration in der wäßrigen Lösung eignet sich Kali- oder Natronlauge, insbesondere 20 - 70 %ige Lauge; vorzugsweise wird 50 %ige Natron- bzw. Kalilauge eingesetzt.

Bei einem trans/cis-Verhältnis von etwa 4 : 1 werden bei diesem Verfahren Gesamtausbeuten um 70 % und darüber erreicht.

Es hat sich weiterhin gezeigt, daß man als Isomeren-Ausgangsmischung unmittelbar die Hydrierlösungen aus einer vorangegangenen Hydrierung von Paracetamol einsetzen kann. Verfahren zur Hydrierung von Paracetamol sind allgemein bekannt und sie liefern in der Regel nahezu quantitativ eine trans/cis-Mischung mit einem trans-Isomerengehalt von mindestens 50 %. Beispielsweise ist aus der Arbeit von Ferber und Brückner (siehe oben) bekannt, daß man mit einem Platinoxidkatalysator in wäßriger Lösung para-Acetaminophenol überwiegend zur trans-Verbindung hydrieren kann. Andere Autoren, wie beispielsweise Billman und Buehler (J.Am.Chem.Soc. 75, 1345, (1953)) bevorzugen alkoholische Lösungen, bei denen beispielsweise Raney-Nickel als Katalysator zum Einsatz kommt. Andere Katalysatoren, die sowohl in wäßrigen als auch in alkoholischen Systemen eingesetzt werden können, sind Katalysatoren auf Platin-, Rhodium-, Ruthenium- oder Palladium-Basis. Pd-, Rh- und Ru-Katalysatoren findet man regelmäßig auf Trägerstoffen vor, wie beispielsweise Al₂O₃ oder Kohlenstoff. Da das erfindungsgemäße Verfahren in wäßriger Lösung durchgeführt wird, bietet es sich an, bei der vorgeschalteten Hydrierung ebenfalls ein wäßriges System einzusetzen. Bei der Auswahl der Hydrierbedingung ist es somit vorteilhaft, einen solchen Katalysator für die Hydrierung zu wählen, der in einem wäßrigen System überwiegend zur Ausbildung der trans-Verbindung führt. Andere Katalysatorsysteme, wie insbesondere auf Ruthenium basierende Systeme, sind nicht bevorzugt, da sie regelmäßig zur cis-Verbindung führen.

Bei der Verwendung einer Hydrierlösung aus einer vorgeschalteten Hydrierung von Paracetamol sollte darauf geachtet werden, daß vor der Abkühlung der Isomerenmischung auf unter 0°C die Hydrolyse der bei der Hydrierung erhaltenen N-(Hydroxycyclohexyl)-acetamide (Acetamidocyclohexanole) vollständig ist. Um dieses zu gewährleisten, hat es sich bewährt, eine die Isomeren enthaltende Lösung in alkalischem Milieu, vorzugsweise unter Rückflußbedingungen, bis zur vollständig abgeschlossenen Hydrolyse zu erwärmen (90 - 120°C). Auf der Hydrolysestufe ist es noch nicht erforderlich, die Lösung durch Zugabe von KOH und/ NaOH so einzustellen, daß der Gefrierpunkt unterhalb oder bei mindestens -10°C liegt. Es ist aber praktikabel, diese Einstellung bereits bei der Hydrolysestufe vorzunehmen.

Gegenstand der Erfindung ist somit weiterhin ein Verfahren zur Herstellung von trans-4-Aminocyclohexanol, bei dem
a) Paracetamol (para-Acetamido-phenol) in an sich bekannter Weise hydriert wird, so daß das erhaltene Produkt (para-Acetamidocyclohexanol) in der Reaktionslösung ein trans/cis-Verhältnis von mindestens 1 : 1 aufweist,
b) nach Abtrennung des Hydrierkatalysators die Reaktionslösung in alkalischem Milieu verseift wird und schließlich
c) unter Abkühlung der Reaktionslösung trans-4-Aminocyclohexanol auskristallisiert wird.

Die in Stufe b) erhaltene Lösung sollte spätestens zu Beginn von Stufe c) derart sein, daß der Gefrierpunkt unterhalb der Temperatur liegt, bei der trans-4-Aminocyclohexanol auskristallisiert. Unter den bevorzugten Verfahrensbedingungen liegt dieser Temperaturwert etwa bei -8°C, so daß die Gefrierpunktserniedrigung etwa bis zu einer Temperatur unterhalb dieser Temperatur eingestellt werden sollte.

Üblicherweise schließen sich weitere Reinigungsschritte an, beispielsweise kann eine azeotrope Entwässerung und Kristallisation aus Toluol durchgeführt werden, um anhängendes Wasser zu entfernen und um das Produkt weiter aufzureinigen. Selbstverständlich können hier auch andere Lösungsmittel eingesetzt werden, wie beispielsweise Ethylether, Dioxan, Petrolether, Ethanol und Benzol.

Die Erfindung wird in den folgenden Beispielen näher erläutert.

### Beispiele

### Hydrierungen von Paracetamol

### Beispiel 1

Es wurden 20 g p-Acetamido-phenol in Gegenwart von 2 g Platinoxid in 1,5 l Wasser über 8 Stunden bei Raumtemperatur in einem Druckgefäß unter einem Wasserstoffdruck von etwa 3 bar hydriert. Nach vollständiger Umsetzung wies die Reaktionslösung ein trans/cis-Verhältnis von etwa 4: 1 auf. Die filtrierte Lösung wurde zur weiteren Verarbeitung zur Seite gestellt.

### Beispiel 2

Es wurden 100 g 4-Acetamido-phenol in ein Hochdruckgefäß eingefüllt und es wurden 250 ml absoluter Alkohol (Ethanol) und 5 g Raney-Nickel zugegeben. Dann wurde das Druckgefäß mit Wasserstoff bis zu einer Druckanzeige von 10 bar beaufschlagt. Im Anschluß daran wurde die Mischung auf 180°C erwärmt und bei dieser Temperatur über 2 Stunden gehalten. Nach Abkühlen auf Raumtemperatur wurde die Lösung filtriert und für die weitere Hydrolyse bereitgestellt. Das trans/cis-Verhältnis war etwa 4 : 1.

### Beispiel 3

Es wurde eine Lösung von 25 g Paracetamol (0,165 Mol) in 150 ml Ethanol über 5 % Rhodium auf Al₂O₃ (etwa 5 g) bei Raumtemperatur und unter einem Wasserdruck von etwa 3,5 bar in einem Druckgefäß hydriert. Die Hydrierung dauerte etwa 4 Stunden. Dann wurde der Katalysator durch Filtration entfernt und mit heißem Ethanol gewaschen (etwa 50 ml). Die vereinigten Filtrate wurden dann für die anschließende Hydrolyse zur Seite gestellt. Das trans/cis-Verhältnis in der Reaktionslösung war etwa 1 : 1.

### Hydrolyse/Isomerentrennung

Es wurden jeweils 100 ml der in den zuvor beschriebenen Beispielen erhaltenen Hydrierlösungen mit so viel 20 %iger Natronlauge versetzt, bis eine deutliche alkalische Reaktion festzustellen war (pH-Wert etwa 13,5). Diese Lösungen wurden in einem 1-Liter-Kolben unter Rückflußbedingungen über 6 bis 8 Stunden erwärmt. Zu der auf Raumtemperatur abgekühlten Lösung wurde im Anschluß daran so viel 50 %ige Natronlauge zugegeben, daß der Gefrierpunkt bei -12°C bis -15°C zu liegen kam. Bei Abkühlung der Lösungen auf -10°C fiel trans-4-Aminophenol aus.

Die zuvor geschilderten Versuche wurden wiederholt, wobei bereits zu Anfang genügend 50 %ige Natronlauge zugegeben wurde, um den pH-Wert auf etwa 14 (oder darüber) einzustellen und den Gefrierpunkt auf -12°C bis -15°C. Insgesamt wurde dabei weniger Natronlauge eingesetzt. Das Volumenverhältnis Hydrierlösung zu Natronlauge war dann etwa 2 : 1 bis 1 : 1.

Die besten Ergebnisse wurden mit der in Beispiel 1 erhaltenen Hydrierlösung erreicht.

### Hydrierung von Paracetamol in technischem Maßstab

In einem Hydrierreaktor wurden 1328 kg (8,8 kmol) Paracetamol vorgelegt und es wurde 3460 l Wasser zugegeben. Das Paracetamol löste sich z. T. auf. Dann wurde mit Natronlauge auf einen etwa neutralen pH-Wert eingestellt. Schließlich wurden 30 kg (1,38 kg Pd) eines Palladium auf Kohle-Katalysators zugegeben (E101 o/w 10 % Pd mit 54 % Wasserfeuchte). Die Hydrierapparatur wurde dann dreimal mit Stickstoff und schließlich mit Wasserstoff gespült. Unter Rühren wurde bei einer Temperatur von 100°C bei einem Wasserstoffdruck von 4,5 bar hydriert. Die Hydrierung wurde über einen Zeitraum von etwa 24 bis 36 Stunden bei einer Temperatur von 98 - 110°C durchgeführt. Die Hydrierung wurde solange fortgesetzt, bis bei einer abgenommenen Analysenprobe kein Paracetamol mehr nachgewiesen werden konnte (GC; Gaschromatographie). Nach beendeter Reaktion ließ man das Reaktionsgemich auf 30 - 40°C abkühlen. Schließlich wurde die erhaltene Hydrierlösung abfiltriert. Der Katalysator kann in die nächste Hydrierung recycliert werden. Das trans/cis-Verhältnis in der Lösung war etwa 80 : 20. Diese Lösung wurde dann in der folgenden Stufe weiterverarbeitet.

### Hydrolyse und Isomerentrennung

Etwa 5150 kg der in dem zuvor beschriebenen Beispiel erhaltenen Hydrierlösung wurden in einen Reaktor eingefüllt. Dieser Lösung wurden etwa 2108 kg Natronlauge (50 %ig) zudosiert. Schließlich brachte man unter Stickstoff das Reaktionsgemisch zum Sieden und hielt die Mischung über 6 Stunden unter Rückflußbedingungen. Die Lösung ließ man dann unter Rühren langsam abkühlen. Dann wurde auf -10°C abgekühlt, wobei ein Produkt auskristallisierte. Der Kristallbrei wurde eine Stunde bei -10°C gerührt. Dann wurde die Suspension abfiltriert und der Feststoff mit 360 l Eiswasser gewaschen. Hierbei erhielt man 1676 kg eines feuchten Rohproduktes (739 kg trocken, Rohausbeute 73 %).

Im Anschluß daran wurde 1032 kg des feuchen Rohproduktes in einem weiteren Reaktor eingeführt, der mit 6600 l Toluol befüllt wurde. Im Anschluß daran wurden Wasser und Toluol azeotrop bei 300 mbar abdestilliert. Das Destillat wurde einer kontinuierlichen Phasentrennung unterworfen und die obere Toluolphase zurückgeführt. Nach vollständiger Abscheidung des Wassers wurde dann die Toluolsuspension bei Normaldruck unter Rückflußbedingungen erhitzt. Nach vollständiger Auflösung des trans-Aminocyclohexanols in dem Reaktor wurden 11 kg Aktivkohle in 20 l Toluol zugegeben. Die Suspension wurde dann eine Stunde unter Rückfluß gekocht und schließlich heiß filtriert. Reaktor und Filter wurden mit 60 1 heißem Toluol nachgespült. Das Filtrat wurde vakuumkristallisiert und anschließend auf 15 -25°C gekühlt. Dann wurden die abfiltrierten Kristalle mit 60 l Toluol (20°C) gewaschen und die erhaltenen Kristalle wurden im Trockenschrank bei 40°C unter Vakuum getrocknet. Hierbei erhielt man insgesamt 410 kg trockenes trans-4-Aminocyclohexanol (analysenrein).

## Patentansprüche

1. Verfahren zur Isomerentrennung einer Mischung aus trans-4-Aminocyclohexanol und cis-4-Aminocyclohexanol, **dadurch gekennzeichnet, daß**
a) eine Isomerenmischung aus trans-4-Aminocyclohexanol und cis-4-Aminocyclohexanol mit einem trans/cis-Verhältnis von mindestens 1 : 1 eingesetzt wird,
b) in einer wäßrigen Lösung dieser Mischung so viel KOH oder NaOH ein- bzw. zugesetzt wird, daß der Gefrierpunkt der wäßrigen Lösung auf mindestens -5°C absinkt, und
c) unter Abkühlung trans-4-Aminocyclohexanol auskristallisiert wird.

2. Verfahren nach Anspruch 1, **dadurch gekennzeichnet, daß** in der wäßrigen Lösung die Konzentration der Aminocyclohexanole mindestens 0,5 Mol/l ist.

3. Verfahren nach Anspruch 1 oder 2, **dadurch gekennzeichnet, daß** die Konzentration der OH⁻-Ionen in der wäßrigen Lösung mindestens 2 Mol/l beträgt.

4. Verfahren nach einem der vorstehenden Ansprüche, **dadurch gekennzeichnet, daß** die Lösung auf -10°C abgekühlt wird.

5. Verfahren nach einem der vorstehenden Ansprüche, **dadurch gekennzeichnet, daß** eine Isomerenmischung mit einem trans/cis-Verhältnis von 3 : 1 bis 4 : 1 eingesetzt wird.

6. Verfahren zur Herstellung von trans-4-Aminocyclohexanol, **dadurch gekennzeichnet, daß**
a) para-Acetamido-phenol in an sich bekannter Weise so hydriert wird, daß das erhaltene Produkt in der Reaktionslösung ein trans/cis-Verhältnis von mindestens 1 : 1 aufweist,
b) nach Abtrennung des Hydrierkatalysators die Reaktionslösung in alkalischem Milieu verseift wird, wobei eine wäßrige Lösung erhalten wird, deren Gefrierpunkt bei mindestens -5°C oder darunter liegt, und
c) unter Kühlung der Reaktionslösung aus Stufe b) trans-4-Aminocyclohexanol ausgefällt wird.

7. Verfahren nach Anspruch 6, **dadurch gekennzeichnet, daß** in Stufe a) in wäßriger Lösung hydriert wird.

8. Verfahren nach Anspruch 6 oder 7, **dadurch gekennzeichnet, daß** die Verseifung bei einer Temperatur von 90 bis 120°C durchgeführt wird.

## Claims

1. Process for the separation of isomers of a mixture of trans-4-aminocyclohexanol and cis-4-aminocyclohexanol, **characterized in that**
a) an isomer mixture of trans-4-aminocyclohexanol and cis-4-aminocyclohexanol having a trans/cis ratio of at least 1:1 is employed,
b) sufficient KOH or NaOH is employed or added into an aqueous solution of this mixture such that the freezing point of the aqueous solution falls to at least -5°C, and
c) trans-4-aminocyclohexanol is crystallized with cooling.

2. Process according to Claim 1, **characterized in that** the concentration of the aminocyclohexanols in the aqueous solution is at least 0.5 mol/l.

3. Process according to Claim 1 or 2, **characterized in that** the concentration of the OH⁻ ions in the aqueous solution is at least 2 mol/l.

4. Process according to one of the preceding claims, **characterized in that** the solution is cooled to -10°C.

5. Process according to any one of the preceding claims, **characterized in that** an isomer mixture having a trans/cis ratio of 3:1 to 4:1 is employed.

6. Process for the preparation of trans-4-aminocyclohexanol, **characterized in that**
a) para-acetamidophenol is hydrogenated in a manner known per se such that the product obtained in the reaction solution has a trans/cis ratio of at least 1:1,
b) after removal of the hydrogenation catalyst the reaction solution is hydrolysed in alkaline medium, an aqueous solution being obtained whose freezing point is at least -5°C or below, and
c) trans-4-aminocyclohexanol is precipitated with cooling of the reaction solution from stage b).

7. Process according to Claim 6, **characterized in that** in stage a) the hydrogenation is carried out in aqueous solution.

8. Process according to Claim 6 or 7, **characterized in that** the hydrolysis is carried out at a temperature of 90 to 120°C.

## Revendications

1. Procédé de séparation des isomères d'un mélange de trans-4-aminocyclohexanol et de cis-4-aminocyclohexanol, **caractérisé en ce que**
a) on utilise un mélange d'isomères de trans-4-aminocyclohexanol et de cis-4-aminocyclohexanol présentant un rapport trans/cis d'au moins 1 : 1,
b) on ajoute à une solution aqueuse de ce mélange suffisamment de KOH ou de NaOH pour que la température de solidification de la solution aqueuse soit abaissée à au moins -5 °C, et
c) on fait cristalliser le trans-4-aminocyclohexanol par refroidissement.

2. Procédé selon la revendication 1, **caractérisé en ce que** la concentration des aminocyclohexanols dans la solution aqueuse est d'au moins 0,5 mole/l.

3. Procédé selon la revendication 1 ou 2, **caractérisé en ce que** la concentration des ions OH⁻ dans la solution aqueuse est d'au moins 2 moles/l.

4. Procédé selon l'une quelconque des revendications précédentes, **caractérisé en ce que** la solution est refroidie à -10 °C.

5. Procédé selon l'une quelconque des revendications précédentes, **caractérisé en ce qu'**on utilise un mélange d'isomères présentant un rapport trans/cis de 3 : 1 à 4 : 1.

6. Procédé de fabrication de trans-4-aminocyclohexanol, **caractérisé en ce que**
a) on hydrogène du para-amino-phénol de façon connue en soi jusqu'à ce que le produit obtenu présente dans la solution de réaction un rapport trans/cis d'au moins 1 : 1,
b) après séparation du catalyseur d'hydrogénation, on saponifie la solution de réaction en milieu alcalin, en obtenant ainsi une solution aqueuse dont la température de solidification est inférieure ou égale à -5 °C, et
c) on fait précipiter le trans-4-aminocyclohexanol de l'étape b) par refroidissement de la solution de réaction.

7. Procédé selon la revendication 6, **caractérisé en ce qu'**on hydrogène en solution aqueuse dans l'étape a).

8. Procédé selon la revendication 6 ou 7, **caractérisé en ce que** la saponification est effectuée à une température de 90 à 120°C.
